(19) Europäisches Patentamt / European Patent Office / Office européen des brevets

(11) **EP 2 314 217 B1**

(12) **EUROPEAN PATENT SPECIFICATION**

(45) Date of publication and mention
of the grant of the patent:
**13.03.2013 Bulletin 2013/11**

(51) Int Cl.:
*G01N 27/02* (2006.01)    *G01N 33/487* (2006.01)

(21) Application number: **09013404.0**

(22) Date of filing: **23.10.2009**

(54) **Method and device for fast measurement of frequency response with scalable short chirp signals**

Verfahren und Vorrichtung zur schnellen Messung des Frequenzgangs mit skalierbaren kurzen Chirp-Signalen

Procédé et dispositif pour la mesure rapide de réponse en fréquence avec des signaux de courte durée balayés en fréquence

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(43) Date of publication of application:
**27.04.2011 Bulletin 2011/17**

(73) Proprietors:
• **Tallinn University of Technology**
**11317 Tallinn (EE)**
• **OÜ Eliko Tehnoloogia Arenduskeskus**
**12618 Tallinn (EE)**

(72) Inventors:
• **Min, Mart**
**13424 Tallinn (EE)**
• **Paavle, Toivo**
**12113 Tallinn (EE)**
• **Land, Raul**
**12611 Tallinn (EE)**
• **Annus, Paul**
**10146 Tallinn (EE)**
• **Parve, Toomas**
**10917 Tallinn (EE)**

(56) References cited:
**WO-A1-2009/096821**

• **TOIVO PAAVLE ET AL: "Using of chirp excitation for bioimpedance estimation: Theoretical aspects and modeling" ELECTRONICS CONFERENCE, 2008. BEC 2008. 11TH INTERNATIONAL BIENNIAL BALTIC, IEEE, PISCATAWAY, NJ, USA, 6 October 2008 (2008-10-06), pages 325-328, XP031352724 ISBN: 978-1-4244-2059-9**
• **PAAVLE T ET AL: "Wideband object identification with rectangular wave chirp excitation" CIRCUIT THEORY AND DESIGN, 2009. ECCTD 2009. EUROPEAN CONFERENCE ON, IEEE, PISCATAWAY, NJ, USA, 23 August 2009 (2009-08-23), pages 421-424, XP031538000 ISBN: 978-1-4244-3896-9**
• **TOIVO PAAVLE ET AL: "Bioimpedance monitoring with improved accuracy using three-level stimulus" CIRCUIT THEORY AND DESIGN, 2007. ECCTD 2007. 18TH EUROPEAN CONFERENCE ON, IEEE, PISCATAWAY, NJ, USA, 27 August 2007 (2007-08-27), pages 412-415, XP031257771 ISBN: 978-1-4244-1341-6**

**Description**

**TECHNICAL FIELD**

The invention belongs to the field of measuring frequency dependant properties of an object, such as bioobject.

**BACKGROUND ART**

Using sine wave excitation and frequency domain measurements, such as bioimpedance measurements is common approach in assessing passive electrical properties of different objects, such as biological matter. However, frequency sweeping or hopping of a sine wave excitation over a wide frequency range is too slow for performing impedance spectroscopy to recover fast impedance changes in biological objects such as single cells and cell cultures in high throughput microfluidic devices. The use of short-time and broad frequency band single-pulse excitation and monitoring the response as a function of time will greatly reduce the measurement interval.

Chirp signals, i.e. signals in which the frequency increases ('up-chirp') or decreases ('down-chirp') continuously as a function of time, are widely used in radar and sonar applications, acoustic, ultrasonic, optical and seismological studies. The main advantage of chirp signals is their well defined frequency range (constant power spectral density, PSD) for wide range of frequencies (from start frequency of the chirp to the stop frequency of the chirp), acceptable crest factor and signal-to-noise ratio. Recently, using chirp and modified chirp signals is proposed for estimation of the frequency response of electrical impedance, particularly the impedance of biological objects (the impedance spectrum), see "Using of Chirp Excitation for Bioimpedance Estimation: Theoretical Aspects and Modeling" by Paavle et al, 2008 International Baltic Conference (BEC2008), October 6-8, 2008. This is considered as closest prior art.

Using of rectangular chirps is also known, see "Bioimpedance Monitoring with Improved Accuracy using Three-Level Stimulus" by Paavle et al, Circuit Theory and Design, 2007. ECCTD 2007. 18th European Conference on, IEEE, Piscataway, NJ, USA, pages 412-415. Signal processing is much simpler for rectangular wave excitation with only constant +A, and -A values. Moreover, the rectangular waveforms have the minimal crest factor (ratio of a peak value to a root-mean-square level) equal to 1. A widely used method is to generate a pseudo-random maximum length sequence (MLS) of rectangular pulses. Another known method is generation of a sequence of regularly shortening or widening rectangular pulses. Also, rectangular chirp signal which can be described as a signum-chirp function instead of the classical sinusoidal chirp, is proposed. Besides the simplest rectangular chirp having non-return-to-zero (NRZ) pulses (zero-states are absent), some versions of return-to-zero (RZ) rectangular pulse chirp function (has +A, 0 and -A values) have been suggested, see "Wideband Object Identification with Rectangular Wave Chirp Excitation" by Paavle et al, Circuit Theory and Design, 2009. ECCTD 2009. European Conference on, 20090823 IEEE, Piscataway, NJ USA - pages 421-424.

Using of chirp signals (both sine wave and rectangular wave based) have several advantages, including short excitation and measurement time, a well determined excitation bandwidth (frequency range), so that the most of generated energy (85 to 99%) is concentrated into the useful bandwidth, and constant or other specified power spectral density (PSD) within the useful bandwidth can be established.

However, the spectroscopy of dynamic objects with rapidly changing impedances is still challenging as it is commonly assumed that the chirps contain hundreds, thousands and at high frequencies even millions of signal cycles. Very fast changing impedances, as in the case of moving objects (as bacteria, cells, droplets, bubbles, etc), require a very short excitation time to avoid the dynamic errors of spectrogram (primarily of the timeline sequence of spectral snapshots) due to the quick changes.

What is needed, therefore, is a fast measurement method scalable in both time and frequency domain for flexible performing of impedance spectroscopy of dynamic impedances.

What is also needed is that as much energy of the signal as possible is generated within the excitation bandwidth as to minimize the power consumption and the heating or other unwanted effect on the object.

**DISCLOSURE OF INVENTION**

The invention is based on a notion that even a short chirp signal having only one cycle has the power spectral density sufficiently similar to a traditional chirp signal with many cycles. Further more, surprisingly even chirp signals as short as one quarter and one half of cycle give satisfactory results in terms of power spectral density.

The goal of the invention is to use such short chirp signals where frequency response from an object must be obtained during very short measurement period. For example, chirp signals with 1, 0.5 or 0.25 cycles (or, 2, 1 and 0.5 half-cycles, respectively) can be used instead of multi-cycle chirps.

According to further embodiment of the invention, the frequency is changed (i.e., starting from the start frequency and increased until the stop frequency for up-chirp, or starting from the start frequency and decreased until the stop frequency for down-chirp) according to any suitable function f=f(t), where t is time, e.g., linearly. However, according to preferred embodiment, the frequency is increased according to an exponential function $m^t$, or according to power law $t^m$, where t is time and m is an arbitrary number.

According to further embodiment of the invention, an amplitude of the chirp signal is also modified (modulated) during the chirp signal. According to one embodiment, the amplitude is increased according to an power function $t^m$. m is selected large enough to straighten the power spectral density for lower frequencies, e.g., from around 2 to around 10 for most cases.

Another goal of the invention is to provide energy efficient signals, i.e. chirp signals with up to 93.5% of useful energy percentage (i.e., the energy within the useful excitation bandwidth).

According to one embodiment of the invention, rectangular chirp (or signum chirp) signals are generated. Particularly energy efficient signals are return-to-zero type rectangular chirp signals.

According to one aspect of the invention, single cycle rectangular chirp signal is provided, where the duration of the cycle $T_{chirp}= 2/f_2$ and the duration of the first half-cycle $T_1$ is $T_{chirp}/2^{1/2}$ and the duration of the second half-cycle $T_2$ is $T_{chirp}-T_1$. If the amplitudes during the first half-cycle and during the second half-cycle are equal, the signal includes DC component. To remove this component, the amplitudes of half-cycles are adjusted so that the areas of the signal in both half-cycles are equal, i.e., the amplitude of the second half-cycle is larger than for the first half-cycle.

Furthermore, the invention allows building wideband scalable spectral analyzers where both excitation time and the frequency range can be set up and changed independently. This allows to conduct fast (but somewhat less accurate) and slower (but more accurate) measurements intermittedly simply by changing the measurement time by choosing an appropriate number of half-cycles within the chirp signal.

The invention is best suitable for wideband and short time spectroscopy of time variant (dynamic) impedances. The chirp signals allow scalability in both frequency and time domain, more than 90% of the generated energy falls into useful bandwidth and rectangular chirp signals allow to reduce the complexity of impedance spectroscopy.

## BRIEF DESCRIPTION OF DRAWINGS

FIG 1 is a 5μs quarter cycle (n=1/2) linear frequency sweep chirp signal for measurement time 20 μs (A) and 200 μs (B) having upper limit of the effective bandwidth $f_2$=100 kHz, and frequency distribution of its relative spectral density (C).

FIG 2 is a 10μs half cycle (n=1) linear frequency sweep chirp signal for measurement time 20 μs (A) 200 μs (B) and having upper effective bandwidth limit $f_2$=100 kHz, and frequency distribution of its relative spectral density (C).

FIG 3 is a 20μs full cycle (n=2) linear frequency sweep chirp signal for measurement time 20 μs (A) 200 μs (B) and having upper effective bandwidth limit $f_2$=100 kHz, and frequency distribution of its relative spectral density (C).

FIG 4 is a 20μs full cycle (n=2) exponential frequency sweep chirp signal for measurement time 20 μs (A) 200 μs (B) and having upper effective bandwidth limit $f_2$=100 kHz, and frequency distribution of its relative spectral density (C).

FIG 5 is a 200μs 10-cycle (n=20) linear frequency sweep chirp signal for measurement time 200 μs having upper limit of the effective bandwidth $f_2$=100 kHz, and frequency distribution of its relative spectral density.

FIG 6 is a 2000μs 100-cycle (n=200) linear frequency sweep chirp signal for measurement time 2000 ps having upper limit of the effective bandwidth $f_2$=100 kHz, and frequency distribution of its relative spectral density.

FIG 7 is a 20μs full (single) cycle (n=2) linear frequency sweep chirp signal with amplitude control by the law of power of 8 for measurement time 20 μs having upper limit of the effective bandwidth $f_2$=100 kHz, and frequency distribution of its relative spectral density.

FIG 8 is a nomogramme of the relationship between the sweep time Ts and upper limit of the effective bandwidth $f_2$ for various values of the number of cycles p in the chirp signal (here p=n/2).

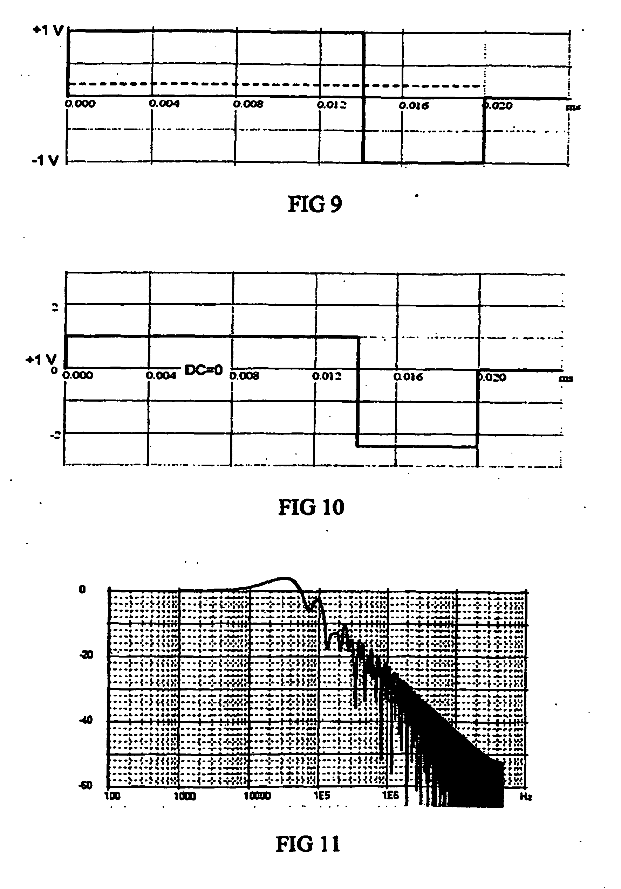

FIG 9 is a minimum length full (single) cycle rectangular chirp; according to first example, the signal includes 0.207 V DC component ($f_1$= 0, $f_2$=100 kHz, Tchirp= 2/f2= 0.02 ms, $T_1$=$T_{chirp}/2^{½}$).

FIG 10 is a minimum length full (single) cycle rectangular chirp, according to second example the DC component of the signal is compensated ($f_2$=100 kHz Tchirp= $2/f_2$= 0.02 ms, $T_1$=$T_{chirp}/2^{1/2}$,$A_1$=1, A2=-[1+sqrt(2)] = -2.4142 V).

FIG 11 is a spectrum of the minimum length single cycle rectangular chirp (Example 2).

FIG 12 is a diagrams of the sine wave chirp, signum of sine (non-return to zero, NRZ) chirp, and rectangular wave return-to-zero (RZ) chirp.

FIG 13 is a dependence of the effectiveness $E_{exc}/E_{ch}$ of the return to zero (RZ) rectangular wave chirp from the duration of the zero-level state part.

FIG 14 is a generalized block diagram of a device for measurement of impedance spectrum using chirp excitation signal.

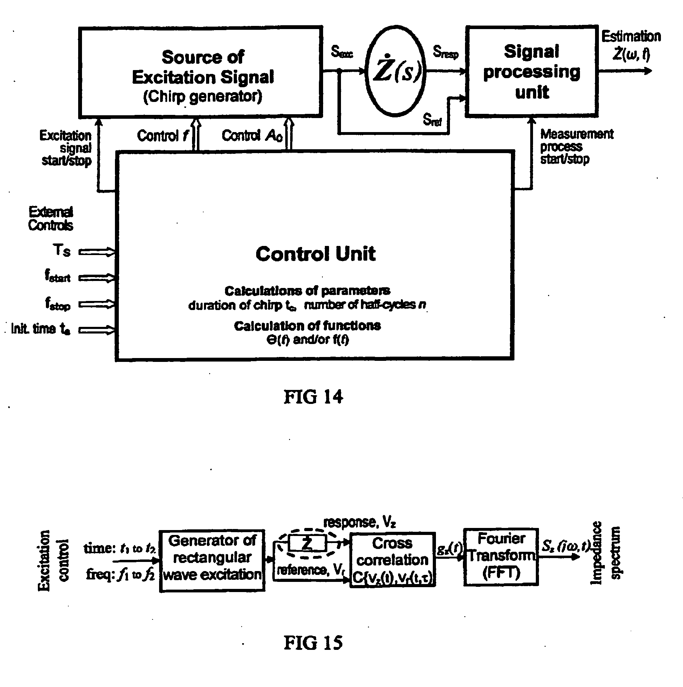

Fig. 15 is a simplified architecture of impedance analyzer, in which the rectangular wave excitation is generated for covering a pre-selected excitation bandwidth $B_{exc}$. The excitation passes the impedance under study $\dot{Z}$ and gives a response, which will be cross-correlated with reference pulses (the normalized excitation having +1 and -1 values, typically). This is a deconvolution process, which results in obtaining the correlation function, which represents the impulse response gz(t). Performing the Fourier transform, we can obtain a complex impedance spectrum, from which we can separate its real and imaginary parts Re{Sz(jω)} and Im{Sz(jω)} or to calculate the frequency responses of magnitude Mz(ω) and phase θz(ω).

FIG 16 is a block diagram of a hardware oriented solution of the fast electrical bioimpedance spectrum measurement system with repeated measurement cycle for the case of low computing resources.

FIG 17 is an extended block diagram of a hardware oriented solution of the fast electrical bioimpedance spectrum measurement system with repeated measurement cycle for the case of low computing resources.

FIG 18 is a reference circuitry for a digital generator of above mentioned rectangular chirps. It contains a reversible shift register (stages QI to Q14), two flip-flops (FF1 and FF2) and some standard logic circuits as NAND, OR and XOR.

FIG 19 is an experimental set-up for performing impedance spectroscopy of droplets in high-throughput microfluidic systems.

## MODES FOR CARRYING OUT THE INVENTION

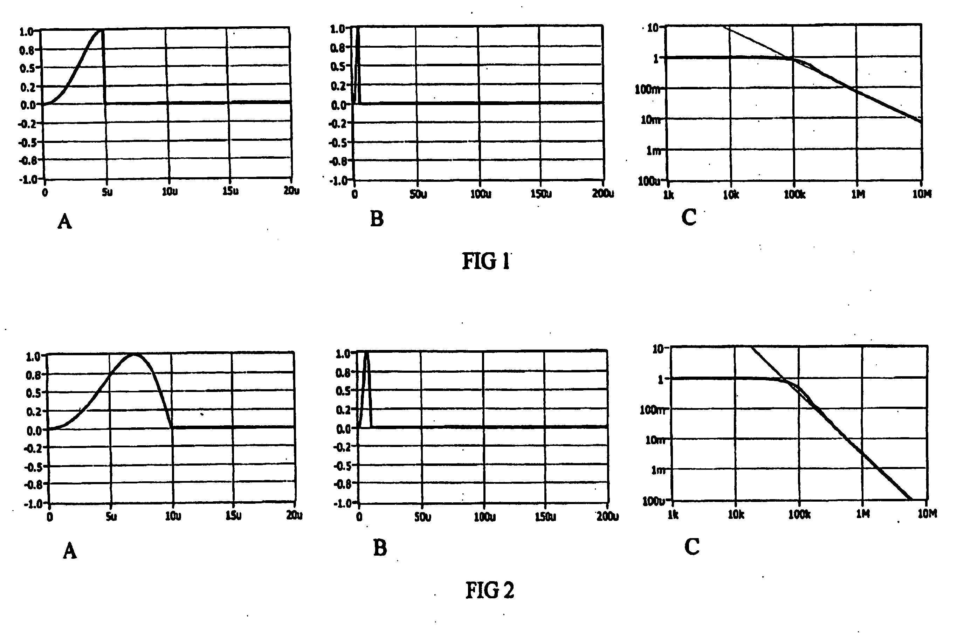

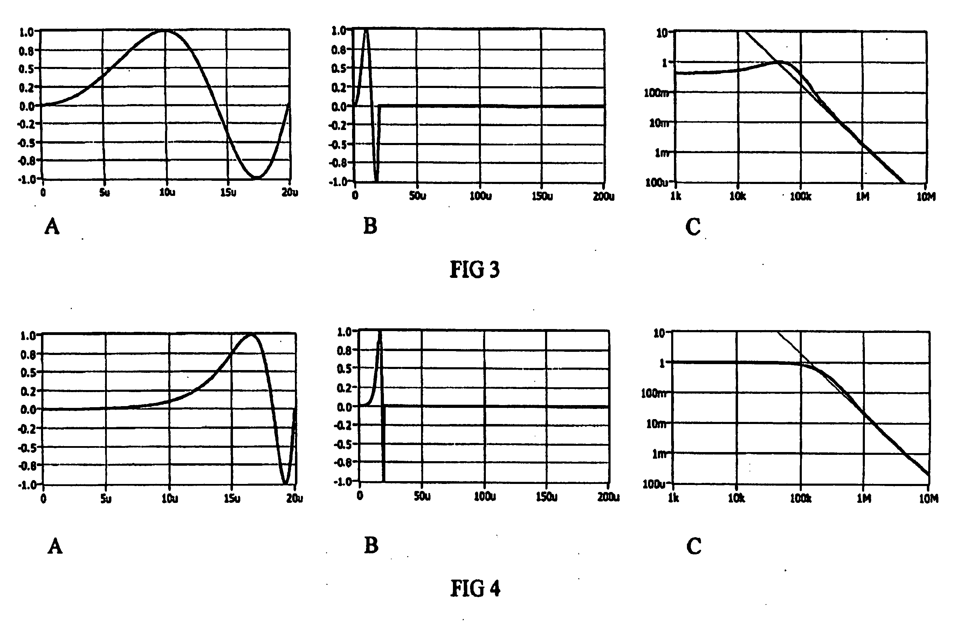

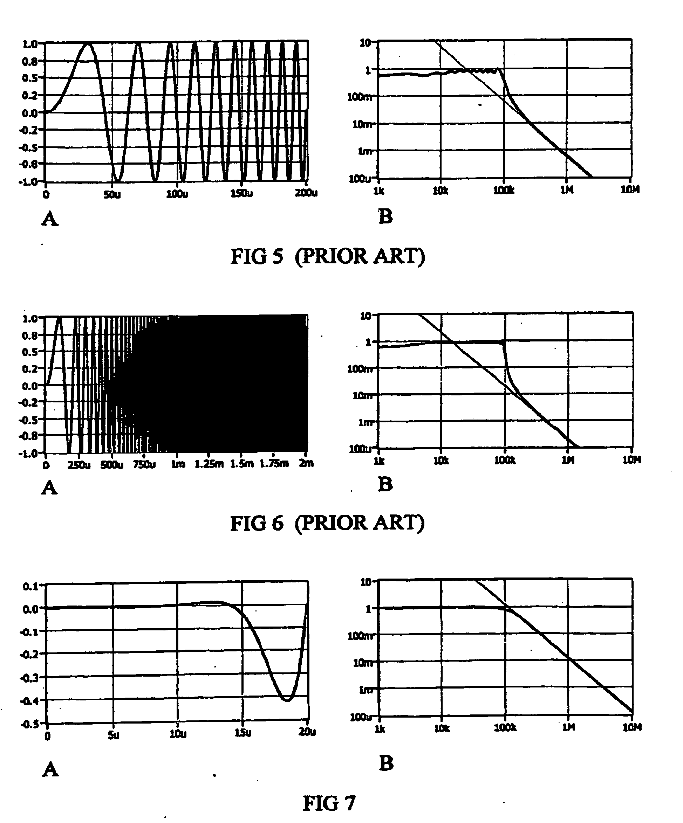

Fig. 15 depicts a simplified architecture of impedance analyzer, in which the chirp signal is generated during the excitation time interval $T_{exc}$ from $t_1$ to $t_2$ for covering a pre-selected excitation bandwidth $B_{exc}$ from $f_1$ to $f_2$. The chirp signal is then introduced into the object under study Z and response signal is then received and used, together with the excitation signal, to calculated the parameters characteristic to the object (such as impedance). For example, a deconvolution process is used, which results in obtaining the correlation function, which represents the impulse response $g_z$(t). Performing now the Fourier transform, a complex impedance spectrum, from which we can separate its real and imaginary parts Re{$S_z$(jω)} and Im{$S_z$(jω)} or to calculate the frequency responses of magnitude $M_z$(ω) and phase $θ_z$(ω).
The chirp signal generator is adjusted to generate a chirp signal with required duration and with required start and stop frequencies. Such generators are known from the art.
In prior art, the chirp signals used are shown on FIG 5A and FIG 6A. Such signals have many, from tens to hundreds to millions of cycles. The duration of such signals (for 100KHz bandwidth) is e.g., from 200µs to 2000µs. Such duration is too long for applications such as microfluidic measurements. The power spectral density of such signals have shown on FIG 5B and FIG 6B correspondingly.
The chirp signals generated and used according to present invention are shown on FIG 1A, FIG 2A, FIG 3A, FIG 4A and FIG 7A. For the same bandwidth (100KHz), the duration of the signal is only from 5µs (FIG 1A), if 1/4 cycle chirp signal is used up to 20µs (FIG 4A) for one cycle chirp. Appears that the power spectral density of such chirp signals is quite similar to much longer chirp signals with many cycles.

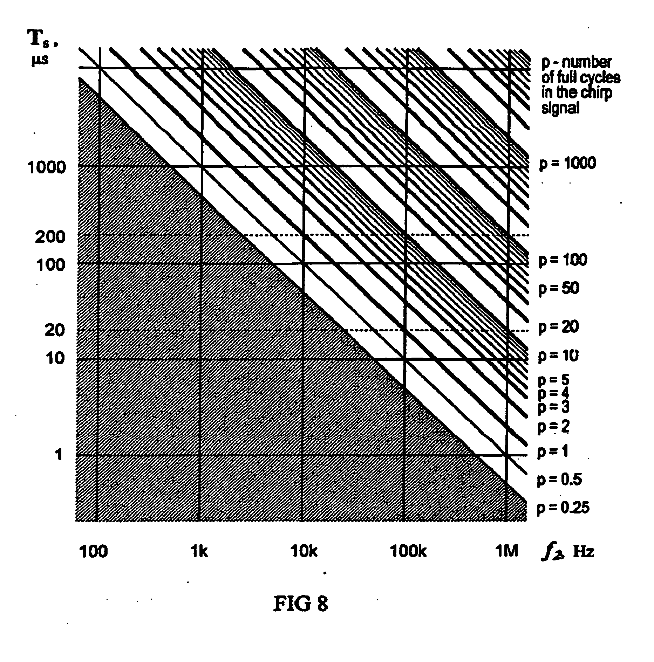

FIG 8 is a nomogramme of the relationship between the sweep time Ts and upper limit of the effective bandwidth $f_2$ for various values of the number of cycles p in the chirp signal (here p=n/2).
The chirp signals with higher p values (p>1000) exhibit similar properties with the conventional sweep signals, the chirp signals with lower p values (p<0.25) exhibit similar properties with the conventional pulse signals and are therefore characterized by corresponding sweep and pulse techniques.

The chirp signals with intermediate p values (p over 0.25 and below 10) exhibit scalable properties and are especially suitable for signal processing techniques in scalable excitation time and frequency range.

While chirp signals with linear increase or decrease of the frequency can be used (FIG 1A to FIG3A), it can be more preferable that the frequency is changed according to different function, for example according to exponential function as shown in FIG 4A.

The signal shown on FIG 7A is further modified by modulating the amplitude of the chirp signal according to appropriate function, i.e. according to a power law $t^{m}$, where *m* is 8.

The advantage of the invention is that the duration of chirp signal can be chosen discretely by half cycles $k\pi$ of the chirp signal, where k is an integer, that is, k = 1, 2, 3, etc, and one full cycle of the chirp signal is $2\pi$. Surprisingly, the shortest chirp can last only one half-cycle. In FIG 1A there is given an example of a half-cycle ($\theta$fin = $\pi$) chirp with the duration of 10 microseconds. As the starting frequency in this example was chosen 1 kHz (at which the period is equal to 1 ms or 1000 $\mu$s), the spectral density of the chirp remains practically constant down to 1 kHz and even to the lower frequencies (see Fig. 1C). The upper limit of the spectrum can be asymptotically quite well presented by a second order asymptote. This crosses the level of the constant spectral density practically at the frequency, which can be found using the duration of the chirp as the half period of the signal at that frequency.

The chirp pulse in Fig. 2A is described mathematically as

$$ch(t) = \sin[2\pi(2B/T)\cdot t^2/2], \qquad\qquad (1)$$

where 0 < t < Texc and duration Texc= T/2 of the chirp pulse (1) is equal to half-cycle of sine wave. The chirp rate B/Texc, Hz/s, corresponds to the excitation bandwidth BW=100kHz (Fig. 2C) covered by the chirp pulse spectrum during one half-cycle T/2 = 10$\mu$s of sine function (1). A bipolar chirp pulse with duration of one full wave cycle T = 20$\mu$s in Fig. 3A has RMS spectrum is shown in Fig. 3C.

Chirp Energy and Impedance Dynamics

An outstanding property of chirp function is that the useful excitation bandwidth BW can be set not dependent on duration of the chirp pulse when choosing appropriate chirp rate B/T$_{exc}$ (1). In Fig. 5A is shown a chirp pulse consisting of 10 full cycles with duration T$_{exc}$= 10T = 200 $\mu$s, the RMS spectrum density of this signal is depicted in Fig. 5B.

Excitation energy depends proportionally on duration of the excitation pulse T$_{exc}$. Therefore, it is reasonable to use longer excitation pulses for obtaining better signal-to-noise ratio. Besides other requirements, the main limiting factor is a speed of impedance variations (dynamics). Thanks to specific properties of the chirp function, it is possible to match the needs for bandwidth, time, signal-to-noise ratio and dynamic requirements when implementing the impedance spectroscopy in lab-on-chip type analyzers and in medical devices.

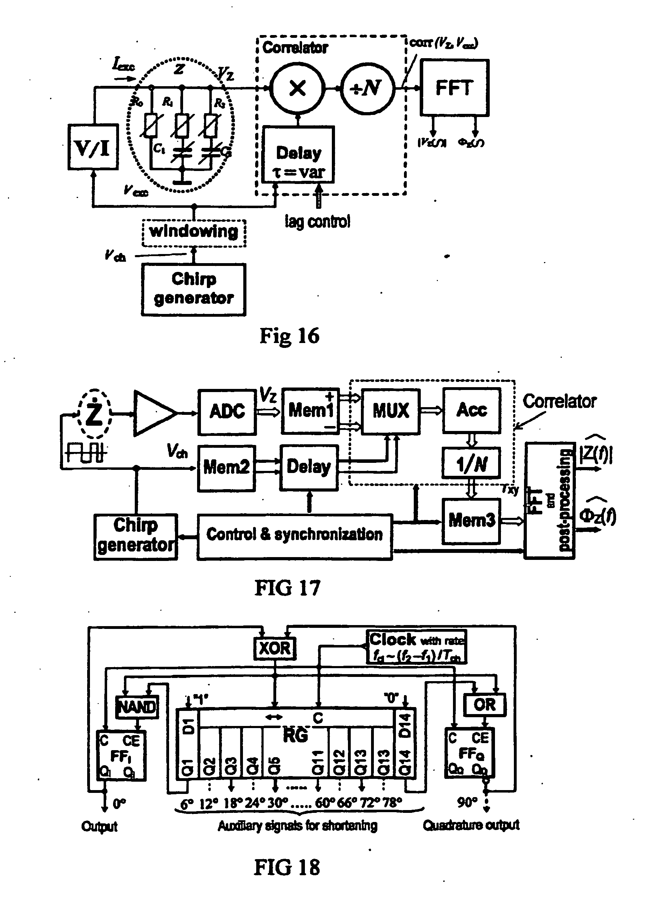

In Fig. 16, the generated chirp pulse V$_{ch}$, passed through an optional window forms the excitation signal V$_{exc}$. Often, a boxcar-type windowing (Tukey window) is used to suppress the Gibbs effect. The test sample is stimulated by the current I$_{exc}$(t) from a voltage-to-current converter V/I. Stimulating causes a response voltage Vz(t), which is attenuated and shifted in phase in relation to the V$_{exc}$ at every instant of time differently, depending on the instant frequency of the chirp.

In FIG 17, the basic structure of a practical evaluation unit is shown, including simplified correlation cell and memory blocks (Mem) for buffering of the V$_{z}$, V$_{ch}$ and r$_{xy}$. Multiplying of the digitized response V$_{z}$ by the lagged $\pm$1 level chirp states is accomplished by multiplexing (MUX) the inversed and non-inversed response values V$_{z}$ stored in the memory Mem1. The products are accumulated (Acc) and averaged for every lag step. The control and signal processing is performed by a FPGA based processor.

In practical experiments the trigger circuit with a central reversible shift register RG (FIG 18) can be applied as the source of the excitation signals. This multiuse circuit can be simply commutated between the generating of NRZ or RZ chirp pulses with the 18° or 30° shortening. The instantaneous frequency of signal is determined by the changing clock rate.

The main advantage of the proposed method is the rapid estimation of complex spectrum of the impedance of biological objects in the wide range of frequencies. The method is also implementable in high throughput microfluidic laboratory-on-chip type devices for performing bioimpedance based joint time-frequency domain analysis of cells, cell cultures and droplets.

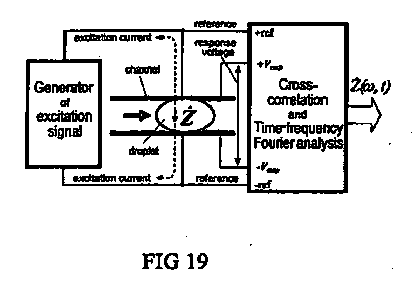

FIG 19 describes a measurement set-up for impedance spectroscopy in microfluidic system. Microfluidic systems comprise a bio-mechanical part and an electronic part forming together a lab-on-a-chip type device. The electronic part generates droplet driving voltages and excitation signals for impedance measurement. It contains a generator of 100 $\mu$A level excitation current with rectangular chirp waveform. The excitation current flows through the droplet under study in a micrometer size microfluidic channel by the aid of current electrodes. The same signal forms also the reference signal for cross-correlating with the response voltage from voltage electrodes. It includes also a signal processing unit, which fulfils cross-correlation and FFT operations for performing the impedance spectroscopy for identifying the properties of cells in the droplet. Droplets with with or without cells will follow after about every 10-100 ms time interval in high-throughput systems. Therefore, the fast impedance measurement in a wide frequency range (e.g. from 1 kHz to 10 MHz) must be performed simultaneously at all required frequencies. Joint time-frequency Fourier transform of the cross-correlation CCF function gives a time dependent complex spectrum Z(jω,t), known also as spectrogram.

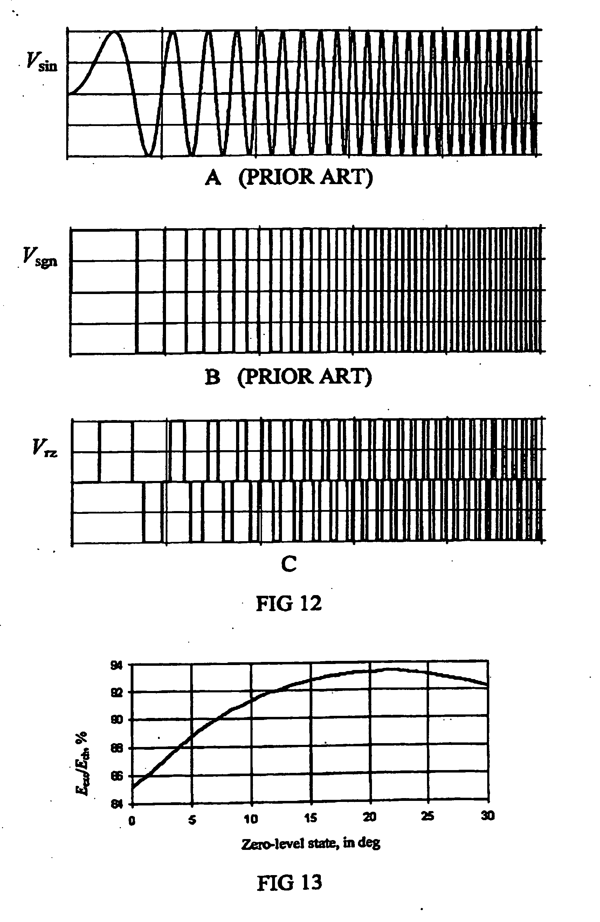

Compared to the sine wave chirp excitation, experiments with the rectangular wave chirps show close results without any significant degradation in measurement accuracy and repeatability of results. But the electronic part is much simpler and less power consuming because of operating only with discrete time 2 or 3-level pulse signals (see FIG 12, B or C).

**Claims**

1. A method for performing impedance spectroscopy by fast measurement of frequency response of biological object having dynamically varying in time parameters, the method comprising:

   determining a frequency range of interest, suitable for characterizing the object;
   determining a maximum measurement time, suitable for measuring the frequency response corresponding to the dynamically varying in time parameters of the object;
   introducing into the object a chirp signal, wherein said chirp signal being a current signal,
   and receiving a response signal from said object and calculating said frequency response from said response signal and said chirp signal, **characterized in that** said chirp signal having a start frequency in one end of the frequency range of interest and a stop frequency in the other end of the frequency range of interest, and having a duration that is shorter or equal to the maximum measurement time, wherein a running frequency of the chirp signal is changing from said start frequency to said stop frequency according to predetermined formula, wherein the number of half-cycles of the chirp signal within the duration of the chirp is less than 4.

2. A method according to claim 1, wherein the number of the half-cycles is 1/2.

3. A method according to claim 1, wherein the number of the half-cycles is 1.

4. A method according to claims 1 to 3, wherein the chirp signal is a sine wave chirp.

5. A method according to claims 1 to 3, wherein the chirp signal is a rectangular wave chirp.

6. A method according to claim 5, wherein the chirp signal is a return-to-zero signal, comprising zero state sections within each half-cycles of the chirp signal.

7. A method according to claim 6, wherein a duration of the zero state sections is from 20 to 25 degrees, preferably from 21.2 degrees to 22.5 degrees, most preferably 21.2 degrees.

8. A method according to claims 1 to 7, wherein the running frequency of the chirp signal is changing linearly from the start frequency to the stop frequency during the duration of the chirp signal so that the number of the half-cycles in the chirp signal is from 1/4 to two.

9. A method according to claims 1 to 7, wherein the running frequency of the chirp signal is changed according to an exponential function $m'$, where $t$ is time and m is an arbitrary number.

10. A method according to claims 1 to 7, wherein the running frequency of the chirp signal is changed according to a power law $t^m$, where $t$ is time and $m$ is an arbitrary number.

11. A method according to claims 1 to 10, wherein an amplitude of the chirp signal is modulated to achieve the most perfect fit of the shape of the power spectrum of excitation chirp to the arbitrary one.

12. A method according to claim 11, wherein the amplitude of the chirp signal is changed according to a power function $t^m$, where t is time and m is arbitrary number.

13. A method according to claim 5, wherein the rectangular chirp is one cycle chirp signal, where the duration of the cycle $T_{chirp}$ is equal to stop frequency divided by 2 and the duration of the first half-cycle $T_1$ is $T_{chirp}/2^{1/2}$ and the duration of the second half-cycle $T_2$ is $T_{chirp} - T_1$.

14. A device for performing impedance spectroscopy by fast measurement of frequency response of a biological object, said device comprising:

a source of chirp signal, adapted to generate a current chirp signal through said biological object; a signal processing unit; a control unit, wherein an output of the source of chirp signal is connected to an object and to a reference input of the signal processing unit, a signal input of which is connected to the object, where the control unit is adapted to form

- the signal of start/stop of the measurement process, which is given to the start/stop inputs of the excitation source and the signal processing unit,
- the signal of chirp frequency control, which is given to the frequency control input of the excitation source, and
- the amplitude control signal, which is given to the amplitude control input of the excitation source, **characterized in that** the source of a chirp signal, adapted to generate a current chirp signal with a number of half-cycles within the duration of the chirp signal from 1/2 to 2.

15. A device according to claim 14, wherein the parameters of the excitation chirp signal including the start/stop interval are calculated in the control unit from the predetermined values for the duration of the excitation chirp signal and the start and stop frequencies or the bandwidth of the sweep, and are used as the constants for forming and calculating the frequency control signal and the amplitude modulation signal.

**Patentansprüche**

1. Eine Methode zur Durchführung der Impedanzspektroskopie durch Schnellmessung des Frequenzgangs eines biologischen Objekts mit zeitlich dynamisch variierenden Parametern, wobei die Methode umfasst:

Bestimmung des Frequenzgangs von Interesse, geeignet zur Charakterisierung des Objekts;
Bestimmung einer maximalen Messzeit, geeignet zur Messung des den zeitlich dynamisch variierenden Parametern entsprechenden Frequenzgangs des Objekts;
Einbringen eines Chirp-Signals in das Objekt, wobei es sich bei besagtem Chirp-Signal um ein Stromsignal handelt, und Empfangen eines Antwortsignals von besagtem Objekt und Berechnung des besagten Frequenzganges aus besagtem Antwortsignal und besagtem Chirp-Signal, welches **dadurch gekennzeichnet, dass** das Chirp-Signal eine Startfrequenz an einem Ende des Frequenzbereichs von Interesse und ein Endsignal am anderen Ende des Frequenzbereichs von Interesse sowie eine Dauer kürzer oder gleich der maximalen Messzeit hat, wobei sich eine Taktfrequenz des Chirp-Signals von besagter Startfrequenz zu besagter Endfrequenz gemäß einer vorbestimmten Formel ändert, wobei die Anzahl der halben Perioden des Chirp-Signals weniger als 4 beträgt.

2. Eine Methode entsprechend Anspruch 1, wobei die Anzahl der halben Perioden 1/2 beträgt.

3. Eine Methode entsprechend Anspruch 1, wobei die Anzahl der halben Perioden 1 beträgt.

4. Eine Methode entsprechend den Ansprüchen 1 bis 3, wobei das Chirp-Signal eine Sinuskurve ist.

5. Eine Methode entsprechend den Ansprüchen 1 bis 3, wobei das Chirp-Signal ein Rechteckwellen-Chirp ist.

6. Eine Methode entsprechend Anspruch 5, wobei das Chirp-Signal ein Zurück-zu-Null-Signal ist, unter Einschluss von Nullzustand-Abschnitten innerhalb jeder halben Periode des Chirp-Signals.

7. Eine Methode entsprechend Anspruch 6, wobei die Dauer der Nullzustand-Abschnitte zwischen 20 und 25 Grad beträgt, vorzugsweise zwischen 21,2 und 22,5 Grad, am besten 21,2 Grad.

8. Eine Methode entsprechend den Ansprüchen 1 bis 7, wobei sich die Taktfrequenz des Chirp-Signals während der Dauer der Chirp-Signals linear von der Startfrequenz zur Endfrequenz verändert, so dass die Anzahl der halben Perioden im Chirp-Signal zwischen 1/4 und zwei beträgt.

9. Eine Methode entsprechend den Ansprüchen 1 bis 7, wobei die Taktfrequenz des Chirp-Signals gemäß einer Exponentialfunktion m' verändert wird, wobei t die Zeit und m eine beliebige Zahl bezeichnet.

10. Eine Methode entsprechend den Ansprüchen 1 bis 7, wobei die Taktfrequenz des Chirp-Signals gemäß einem Potenzgesetz $t^m$ verändert wird, wobei t die Zeit und m eine beliebige Zahl bezeichnet.

11. Eine Methode entsprechend den Ansprüchen 1 bis 10, wobei die Amplitude des Chirp-Signals moduliert wird, um eine möglichst perfekte Anlassung der Gestalt des Leistungsspektrums des Anregungs-Chirps an einen beliebigen zu erzielen.

12. Eine Methode entsprechend Anspruch 11, wobei die Amplitude des Chirp-Signals gemäß einer Potenzfunktion $t^m$ verändert wird, wobei t die Zeit und m eine beliebige Zahl bezeichnet.

13. Eine Methode entsprechend Anspruch 5, wobei es sich beim rechteckigen Chirp um ein einperiodiges Chirp-Signal handelt, wobei die Dauer der Periode $T_{chirp}$ gleich der Ezadfrequenz geteilt durch 2, die Dauer der ersten halben Periode $T_1$ $T_{chirp}/2^{1/2}$ und die Dauer der zweiten halben Periode $T_2$ $T_{chirp}$ - $T_1$ beträgt.

14. Ein Gerät zur Durchführung der Impedanzspektroskopie durch Schnellmessung des Frequenzganges eines biologischen Objekts, wobei besagtes Gerät umfasst:

Die Quelle eines Chirp-Signals, angepasst, um ein Strom-Chirp-Signal durch besagtes biologisches Objekt zu erzeugen; eine Signalverarbeitungseinheit; eine Steuerungseinheit, wobei die Ausgabe der Quelle des Chirp-Signals mit einem Objekt und einer Referenzeingabe der Signalverarbeitungseinheit verbunden ist, deren Signaleingabe mit dem Objekt verbunden ist, wobei die Steuerungseinheit angepasst wird, um

- das Signal zum Beginn/Ende des Messprozesses, welches an die Start/Stopp-Eingabe der Anregungsquelle und die Signalverarbeitungseinheit gegeben wird,
- das Signal zur Kontrolle der Chirp-Frequenz, welches an die Frequenzkontroll-Eingabe der Anregungsquelle gegeben wird, und
- das Amplitudenkontrollsignal, welches an die Amplitudenkontrolleingabe der Anregungsquelle gegeben wird, **dadurch gekennzeichnet, dass** die Quelle des Chirp-Signals angepasst wird, um ein Strom-Chirp-Signal mit einer Anzahl halber Perioden innerhalb der Dauer des Chirp-Signals zwischen 1/2 und 2 zu erzeugen, zu formen.

15. Ein Gerät entsprechen Anspruch 14, wobei die Parameter des Anregungs-Chirp-Signals einschließlich des Start/Stopp-Intervalls in der Kontrolleinheit aus vorbestimmten Werten für die Dauer des Anregungs-Chirp-Signals und der Start- und Endfrequenzen oder der Sweep-Bandbreite berechnet werden, und als Konstanten zur Formung und Berechnung des Frequenzkontrollsignals und des Amplitudemnodulationssignals verwendet werden.

## Revendications

1. Une méthode permettant de réaliser une spectroscopie d'impédance par mesure rapide de la réponse en fréquence d'un objet biologique possédant des paramètres variant dynamiquement dans le temps, la méthode comprenant:

la détermination d'une plage de fréquence d'intérêt, appropriée pour la caractérisation de l'objet;
la détermination d'un temps de mesure maximal, adapté pour la mesure de la réponse en fréquence correspondante aux paramètres variant dynamiquement dans le temps de l'objet;
l'introduction dans l'objet d'un signal chirp où ledit signal chirp est un signal de courant, et la réception d'un signal de réponse dudit objet, ainsi que le calcul de la réponse en fréquence à partir dudit signal de réponse et dudit signal chirp, **caractérisée en ce que** ledit signal chirp possède une fréquence de départ à une extrémité de la plage de fréquences d'intérêt et une fréquence d'arrêt à l'autre extrémité de la plage de fréquences d'intérêt, et a une durée inférieure ou égale au temps maximal de mesure, où une fréquence variable du signal chirp change de ladite fréquence de départ à ladite fréquence d'arrêt, selon la formule prédéterminée, où le nombre de demi-cycles du signal chirp pendant la durée du signal chirp est inférieur à 4.

**2.** Une méthode conforme à la revendication 1, où le nombre de demi-cycles est 1/2.

**3.** Une méthode conforme à la revendication 1, où le nombre de demi-cycles est 1.

**4.** Une méthode conforme aux revendications 1 à 3, où le signal chirp est un signal chirp sinusoïdal.

**5.** Une méthode conforme aux revendications 1 à 3, où le signal chirp est un signal chirp rectangulaire.

**6.** Une méthode conforme à la revendication 5, où le signal chirp est un signal de retour à zéro, comprenant des passages à l'état zéro à chaque demi-cycle du signal chirp.

**7.** Une méthode conforme à la revendication 6, où la durée des états zéro est de 20 à 25 degrés de phase, de préférence de 21.2 degrés à 22.5 degrés, l'idéal étant 21.2 degrés.

**8.** Une méthode conforme aux revendications 1 à 7, où la fréquence variable du signal chirp change linéairement à partir de la fréquence de début jusqu'à la fréquence de fin pendant la durée du signal chirp afin que le nombre de demi-périodes du signal chirp soit compris entre 1/4 et deux.

**9.** Une méthode conforme aux revendications 1 à 7, où la fréquence variable du signal chirp est changée selon une fonction exponentielle $m'$, où $t$ est le temps et m est un nombre quelconque.

**10.** Une méthode conforme aux revendications 1 à 7, où la fréquence variable du signal chirp est changé selon une loi de puissance $t^m$, où $t$ est le temps et $m$ est un nombre quelconque.

**11.** Une méthode conforme aux revendications 1 à 10, où l'amplitude du signal chirp est modulée afin de réaliser le meilleur ajustement possible de la forme du spectre de puissance du signal chirp d'excitation.

**12.** Une méthode conforme à la revendication 11, où l'amplitude du signal chirp est changée selon une fonction de puissance $t^m$, où t est le temps et m est un nombre quelconque.

**13.** Une méthode conforme à la revendication 5, où le signal chirp rectangulaire est un signal chirp d'une période, où la durée de la période $T_{chirp}$ est égale à la fréquence d'arrêt divisée par 2, la durée de la première demi-période $T_1$ est $T_{chirp}/2^{1/2}$ et la durée de la seconde demi-période $T_2$ est $T_{chirp}$ - - $T_1$.

**14.** Un appareil réalisant une spectroscopie d'impédance par mesure rapide de la réponse en fréquence d'un objet biologique, ledit appareil comprenant:

    - un générateur de signal chirp, adapté à la génération d'un signal chirp à travers ledit objet biologique; une unité de traitement du signal; une unité de contrôle, où une sortie du générateur de signal chirp est connectée à un objet et à une entrée de référence de l'unité de traitement du signal, une entrée de cette unité est connectée à l'objet, où l'unité de contrôle permet de créer
    - le signal de début/fin du processus de mesure, qui est donné aux entrées start/stop de la source d'excitation et de l'unité de traitement du signal,
    - le signal de contrôle de la fréquence du signal chirp, qui est donné à l'entrée de contrôle de la fréquence de la source d'excitation, et
    - le signal de contrôle de l'amplitude, qui est fourni à l'entrée de contrôle de l'amplitude de la source d'excitation **caractérisée en ce que** la source du signal chirp, est adaptée à générer un signal chirp de courant avec un nombre de demi-cycles pendant la durée du signal chirp de 1/2 à 2.

**15.** Un appareil conforme à la revendication 14, où les paramètres du signal chirp d'excitation incluant l'intervalle de temps entre début et arrêt sont calculés dans l'unité de contrôle à partir des valeurs prédéterminées pour la durée du signal chirp d'excitation et les fréquences de début et d'arrêt ou la bande passante du balayage et sont utilisées comme constantes pour générer et calculer le signal de contrôle de la fréquence et le signal de modulation de l'amplitude.

FIG 1

FIG 2

A

B

C

FIG 3

A

B

C

FIG 4

EP 2 314 217 B1

FIG 5 (PRIOR ART)

FIG 6 (PRIOR ART)

FIG 7

FIG 8

**FIG 9**

**FIG 10**

**FIG 11**

$V_{sin}$

A (PRIOR ART)

$V_{sgn}$

B (PRIOR ART)

$V_{rz}$

C

FIG 12

$E_{cu}/E_{ch}$ %

94
92
90
88
86
84

0    5    10    15    20    25    30

Zero-level state, in deg

FIG 13

**Source of Excitation Signal (Chirp generator)**

$S_{exc}$

$\dot{Z}(s)$

$S_{resp}$

**Signal processing unit**

Estimation $Z(\omega, f)$

$S_{ref}$

Excitation signal start/stop

Control $f$

Control $A_0$

Measurement process start/stop

External Controls

$T_s$

$f_{start}$

$f_{stop}$

Init. time $t_e$

**Control Unit**

**Calculations of parameters**
duration of chirp $t_c$, number of half-cycles $n$

**Calculation of functions**
$\Theta(f)$ and/or $f(t)$

**FIG 14**

Excitation control

time: $t_1$ to $t_2$

freq: $f_1$ to $f_2$

**Generator of rectangular wave excitation**

response, $V_z$

$Z$

reference, $V_r$

**Cross correlation** $C\{V_z(t), V_r(t,\tau)\}$

$g_z(t)$

**Fourier Transform (FFT)**

$S_z(j\omega, t)$

Impedance spectrum

**FIG 15**

**Fig 16**

**FIG 17**

**FIG 18**

**FIG 19**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PAAVLE et al.** Using of Chirp Excitation for Bioimpedance Estimation: Theoretical Aspects and Modeling. *International Baltic Conference (BEC2008,* 06 October 2008
- **PAAVLE et al.** Bioimpedance Monitoring with Improved Accuracy using Three-Level Stimulus. *Circuit Theory and Design, 2007. ECCTD 2007. 18th European Conference on, IEEE,* 2007, 412-415

- **PAAVLE et al.** Wideband Object Identification with Rectangular Wave Chirp Excitation. *Circuit Theory and Design, 2009. ECCTD 2009. European Conference on, 20090823 IEEE,* 23 August 2009, 421-424